# EUROPEAN PATENT APPLICATION

(11) **EP 1 787 971 A2**
(43) Date of publication of application: **23.05.2007**
(21) Application number: 07004664.4
(22) Date of filing: 25.07.2003
(51) Int. Cl.: C07C 43/215, C07C 41/16, C07B 41/04, C07D 301/03, C07D 303/27

(54) **Process for preparing aryl allyl ethers**

(30) Priority: 02.08.2002 US 210905
(62) Divisional of application: 03766924.9
(71) Applicant: Dow Global Technologies Inc., Midland, MI 48674 (US)
(72) Inventor: Boriack, Clinton J., Brenham Texas 77834 (US); Liao, Zeng K., Lake Jackson TX 77566 (US)
(74) Representative: Casalonga, Axel

(57) **Abstract**

A process for preparing an aryl allyl ether is disclosed comprising reacting (a) a phenolic compound with (b) an allyl acetate in the presence of (c) an allylation catalyst selected from a ruthenium or rare earth metal catalyst complex comprising at least one stable ligand containing a substituted aromatic-containing moiety, wherein the aromatic-containing ligand is cyclopentadienyl (Cp), pentamethylcyclopentadienyl (Cp*), indenyl (In), or 9-fluorenyl (F1), or a substituted olefinic-containing ligand, wherein the olefinic moiety is a cyclooctadienyl (COD) or butyldienyl moiety or substituted olefinic-containing ligands. A process for the preparation of an epoxy-containing compound is disclosed comprising epoxidizing the aryl allyl ether compound made according to this invention.

## Description

The present invention relates to a process for preparing aryl allyl ether compounds by reacting a phenolic compound with an allylating agent in the presence of a transition metal or rare earth metal catalyst complexed with a strongly bonded, non-replaceable stable ligand. The ligand of the transition metal or rare earth metal catalyst complex may be (i) an olefinic-containing ligand or an aromatic-containing ligand; or (ii) a polymeric ligand or a heteroatom-containing multidentate ligand. Aryl allyl ether compounds are useful in preparing epoxy-containing compounds.

A well-known industrial process for producing epoxy resins on a large commercial scale is a process comprising the steps of:
(I) reacting an active hydrogen-containing compound such as an alcohol, a phenol, a thiol, a carboxylic acid, or an amine with an epihalohydrin, such as epichlorohydrin (ECH) or epibromohydrin, to make an α-halohydrin as a reactive intermediate; and
(II) converting the α-halohydrin intermediate of Step (I) into a glycidyl ether, glycidyl thioether, glycidyl ester, or glycidyl amine under basic reaction conditions.

The most widely made and particularly useful epoxy resin is bisphenol A (bis A) epoxy resin which is made by first conducting a coupling reaction of bis A and ECH to form a bis(α-chlorohydrin) intermediate (generally described in Step (I) above); and then, converting the bis A bis(α-chlorohydrin) intermediate to a bis A diglycidyl ether epoxy resin in an epoxide ring-forming dehydrochlorination reaction with a base (generally described in Step II above). Such a two-step process for making an epoxy resin from ECH is described by H. Lee and K. Neville in Handbook of Epoxy Resins, McGraw-Hill Book Co., New York, New York, 1982, Reissue, 2-3 to 2-4.

The above two-step process of coupling bis A and ECH followed by epoxide ring-forming dehydrochlorination has also previously been combined into a single-step reaction, wherein the bis(α-chlorohydrin) intermediate of bis A is generated *in situ* and converted into an a bis A diglycidyl ether epoxy resin simultaneously. Such a single-step process for making bis A epoxy resin is described in U.S. Patent Nos. 4,499,255; 4,778,863; and 5,028,686.

Although ECH is an important commercial product for making the α-chlorohydrin intermediates for bis A epoxy resins, and epoxy resins in general, ECH provides a chlorine-intensive route to producing epoxy resins. In the predominate commercial process for making ECH, ECH is made from allyl chloride, and allyl chloride is made from thermal chlorination of propylene using chlorine gas, a process that. produces chlorinated by-products. Generally, chlorinated by-products are treated as waste material.

Additionally, ECH provides a process water-intensive route to producing epoxy resins. A large amount of process water is used in the reaction of allyl chloride with additional chlorine to make the ECH intermediate, propylene dichlorohydrin intermediate. This process water must eventually be treated as waste.

Therefore, from an environmental standpoint, there is a desire to replace the use of ECH in the production of epoxy resin; and thus, reduce the consumption of chlorine and reduce the generation of chlorinated by-products and reduce the generation of waste water in the production of epoxy resin.

In addition, epoxy resins made from ECH may have a high organic chloride content which may be deemed as undesirable in some applications, for example, in electronic applications.

Because of the aforementioned disadvantages in using ECH for manufacturing epoxy resins, it is desired to provide a non-ECH process for making epoxy resins. That is, it is desired to provide an alternative epoxy resin route, *that is,* an alternative process without using ECH for manufacturing epoxy resins such as bis A epoxy resin.

One non-ECH process as an alternative to the conventional ECH process is disclosed in U.S. Patent No. 5,578,740. The process described in U.S. Patent No. 5,578,740 includes a process to make an epoxy resin comprising the steps of:
(1) providing an allylation reagent which is substantially halogen-free and which reacts with a hydroxyl, a thiol, a carboxylic acid, or amine group to form an allyl ether, allyl thioether, allyl ester or allyl amine, respectively;
(2) allylating one or more compounds containing at least one active hydrogen atom using the allylation reagent from Step (1), whereby allyl ether, allyl thioether, allyl ester or allyl amine compound is formed; and
(3) converting the allyl groups present in the compound from Step (2) to epoxide groups, whereby a glycidyl ether, glycidyl thioether, glycidyl ester or glycidyl amine compound is formed.

More specifically, the efficient, high yield production of epoxy-containing compounds, in three steps starting from the basic raw material, propylene is described in U.S. Patent No. 5,578,740. While the process of U.S. Patent No. 5,578,740 describes a three-step process for production of epoxy containing compounds starting from propylene, other prior art processes involve four or more steps starting from the basic raw material propylene. The three-step process of U.S. Patent No. 5,578,740 utilizing propylene may be summarized as follows:
(1) Propylene + oxygen + acetic acid + catalyst → allyl acetate (allylation reagent);
(2) Allyl acetate + active hydrogen-containing compound such as bisphenol A → diallyl derivative of active hydrogen-containing compound, such as the diallyl ether of bisphenol A; and
(3) Diallyl derivative of active hydrogen-containing compound + peroxy oxidant → diglycidyl derivative of active hydrogen-containing compound, such as the diglycidyl ether of bisphenol A.

When compared with the aforementioned well-known industrial process for producing epoxy resins, the process of U.S. Patent No. 5,578,740 to make epoxy resins has the following advantages: fewer process steps are required when starting with propylene; a non-halogen process is used, thus, there are no halogenated by-products associated with production of epichlorohydrin to be treated as waste and there are no halogenated contaminants, or by-products, to adversely impact the properties of the resulting epoxy resin; and there are no large amounts of process water associated with the production of epichlorohydrin to be treated as waste.

Step (1) of the process described in U.S. Patent No. 5,578,740 provides for an allylation reagent which is substantially halogen-free and which reacts with a hydroxyl, a thiol, a carboxylic acid, or an amine group to form an allyl ether, allyl thioether, allyl ester or allyl amine, respectively. Such allylation reagents generally fall into one of two types: (a) allyl esters, methallyl esters, or higher substituted allyl esters of carboxylic acids; and (b) allyl carbonates, methallyl carbonates, or higher substituted allyl carbonates. The carbonates may be symmetrical such as diallyl carbonate, but are more generally mixed carbonates such as allyl methyl carbonate.

Step (2) of the process described in U.S. Patent No. 5,578,740 provides for allylating one or more compounds containing at least one active hydrogen atom using the allylation reagent from Step (1), whereby an allyl ether, allyl thioether, allyl ester, or allyl amine compound is formed. The allylation process of Step (2) is generally carried out in the presence of a transition metal catalyst and optionally with a basic acting compound which is present in catalytic to greater than stoichiometric amounts. The conditions for the allylation reaction are generally dependent on the type of the allylation reagent being used in the allylation reaction.

Allylation of active hydrogen-containing compounds with type (a) allylation reagents including allyl esters, methallyl esters, or higher substituted allyl esters of carboxylic acids, is carried out in the presence of a transition metal catalyst and usually in the presence of a basic acting material. For example, the allylation of the active hydrogen-containing compound methanol with a substituted allyl acetate allylation reagent with molybdenum and tungsten catalysts has been reported by A. V. Malkov, et. al, in J. Org. Chem., 64, pp. 2737-2750, (1999). The allylation of active hydrogen-containing thiol compounds with allyl acetate using ruthenium catalysts is discussed by T. A. Mitsudo, et. al, in J. Am. Chem. Soc., 121, (37), pp. 8657-8658, (1999). The allylation of the active hydrogen-containing compound phenol with the allylation reagent allyl acetate with palladium catalysts has been reported, for example, by K. Takahashi, et. al, in Bull. Chem. Soc. Japan, 45, pp. 230-236, (1972) and by J. Muzart, et. al, in J. Organomet. Chem., 326, pp. C23-C28, (1987). In these last two cited references, the palladium catalyst efficiencies (catalyst efficiency is defined as turn over number, "TON") calculated on a per hour basis are extremely low, 8 TON and 1 TON, respectively.

Palladium catalyzed allylation of active hydrogen-containing phenolic compounds using allyl esters of carboxylic acids and allyl carbonates is also disclosed in U.S. Patent No. 5,578,740. U.S. Patent No. 5,578,740 discloses that transition metal catalyst efficiency can be 12,800 TON per hour by carrying out the allylation reaction in a two-phase reaction including an aqueous phase in which the pH of the aqueous phase is carefully controlled between 9 to 12. In this pH range, a carboxylic acid salt of the corresponding allyl carboxylate is the by-product of this allylation process. Unfortunately, carboxylic acid salts, such as sodium acetate formed when allyl acetate is used as the allylating agent, are not economically recoverable as a free carboxylic acid, such as acetic acid, which can be recycled to manufacture an allyl carboxylic acid ester, such as allyl acetate, and thus, the carboxylic acid salts must be disposed of as a waste material. It would be desirable to provide an alternative, non-carboxylic acid salt forming process, to the process described in U.S. Patent No. 5,578,740.

Allylation of active hydrogen-containing compounds with type (b) allylation reagents including allyl carbonates, methallyl carbonates, or higher substituted allyl carbonates, is carried out in the presence of transition metal catalysts. Allylation with carbonates generally does not require a basic acting compound, but in some instances basic additives may be used. For example, the allylation of the active hydrogen-containing compound phenol with allyl methyl carbonate allylation reagent with nickel catalysts has been described by A. Mortreux, et. al, in J. Chem. Soc., Chem. Commun., pp.1863-1864, (1995) . The allylation of the active hydrogen-containing compound bisphenol A with allyl methyl carbonate allylation reagent with palladium catalysts has been described in U.S. Patent No. 4,507,492 and by S. Sivaram and A. G. Shaikh in Macromolecular Reports, A32(Suppl. 7), pp. 1053-1060, (1995). As allylation reagents, allyl carbonates provide the opportunity for easily recoverable and recyclable by-products, such as methanol and carbon dioxide, from the allylation reaction using allyl methyl carbonate. However, unfortunately, allyl carbonates are expensive and not readily available on a commercial scale.

In addition to the two aforementioned allylation reagents, there is a third type (c) allylation reagent that is a much less efficient allylating agent than the aforementioned allyl esters and allyl carbonates than can be provided in Step (1) of the process described in U.S. Patent No. 5,578,740. The type (c) allylation reagent, comprises allyl alcohol, methallyl alcohol, or higher substituted allyl alcohols.

Allylation of active hydrogen-containing compounds with type (c) allylation reagents including allyl alcohol, methallyl alcohol, or higher substituted allyl alcohols, is carried out in the presence of transition metal catalysts and generally without the use of basic acting compounds. For example, the allylation of the active hydrogen-containing compounds phenol, trimethyl phenol, and bisphenol A with allyl alcohol has been reported in low yields (for example, an 18.2 percent yield of the allyl ether of bisphenol A) using palladium as the catalyst in Japanese Patent No. 5306246. Yang, et. al, in Organometallics, (20), pp. 5326-5330, (2001) report that titanium cocatalysts are useful in the allylation of aromatic amines with allyl alcohol. The allylation of active hydrogen-containing thiol compounds with allyl alcohol using ruthenium catalysts is discussed by T. A. Mitsudo, et. al, in J. Am. Chem. Soc., 121, (37), pp. 8657-8658, (1999) . Ruthenium transition metal catalysts have also recently been observed by E. Bouwman, et. al, in J. Mol. Catal. A: Chem., 159, pp. 163-177, (2000), as catalysts for allylation of monophenols with allyl alcohol allylation reagent. However, it has been reported by E. Bouwman, et. al, in J. Organometallic Chem., 650, pp. 1-24, (2002), that with the use of allyl alcohol as the allyation reagent, the major products formed from allyl alcohol in the reaction are the homocoupled, undesired product, diallyl ether and ketones from alcohol isomerization, and the desired, crosscoupled allyl phenyl ethers are the minor products. Although allyl alcohol is commercially available, it is relatively expensive compared to allyl acetate, and the catalytic processes using allyl alcohol for allylation are inefficient in selectivity to the desired product.

In view of the disadvantages of the prior known allylation processes described above, it would be desirable: (A) to provide an alternative process for allylation of active hydrogen-containing compounds or mixtures of active hydrogen-containing compounds, particularly phenols or mixture of phenols, using an inexpensive and readily available allylation material, such as an allyl ester of a carboxylic acid as the allylation reagent; and (B) to provide an alternative process for allylation of active hydrogen-containing compounds or mixtures of active hydrogen-containing compounds, in particular phenols or mixture of phenols, using a transition metal or rare earth metal catalyst without the use of a basic acting material, so the allylation can be done in a single phase under neutral to acidic pH; therefore, allowing recovery of the carboxylic acid by-product from the allylation reaction.

The present invention is directed to a process for preparing an aryl allyl ether comprising reacting: (a) a phenolic compound or mixture of phenolic compounds with (b) an allylating agent such as an allyl carboxylate or an allyl carbonate in the presence of (c) a transition metal or rare earth metal catalyst complexed with at least one strongly bonded, non-replaceable stable ligand whereby an aryl allyl ether is formed. The ligand of the transition metal or rare earth metal catalyst complex may be (i) an olefinic-containing ligand or an aromatic-containing ligand; or (ii) a polymeric ligand or a heteroatom-containing multidentate ligand.

In one embodiment of the present invention, the transition metal or rare earth metal catalyst used in the present invention may be a particular catalyst selected from the group of transition metals consisting of ruthenium, rhenium, molybdenum, tungsten, rhodium, palladium, iridium, platinum, nickel, cobalt, or iron; or selected from the group of rare earth metals consisting of lanthnum, ytterbium, or samarium; or any combination thereof either as the metal with organic ligand(s) or as the metal salt.

In another specific embodiment of the process of the present invention, allyl acetate, for example, may be used for allylation of phenolic compounds, without the need of using a base or a basic buffer in the process, in the presence of a ruthenium, iridium or palladium catalyst having at least one strongly bonded, non-replaceable stable ligand whereby an aryl allyl ether is formed. The ligand of the transition metal or rare earth metal catalyst complex may be (i) an olefinic-containing ligand or an aromatic-containing ligand; or (ii) a polymeric ligand or a heteroatom-containing multidentate ligand. In such a process of the present invention, acetic acid, for example, is formed as the by-product of the allyl acetate allylation reaction instead of an acetate salt. Therefore, the recovery and the recycle of acetic acid becomes feasible using the process of the present invention.

Generally, the process of the present invention is directed to preparing an aryl allyl ether compound by reacting a phenolic compound with an allylating reagent such as for example an allyl carboxylate or allyl carbonate as the allylating reagent in the presence of a transition metal or rare earth metal catalyst complexed with at least one strongly bonded, non-replaceable stable ligand whereby an aryl allyl ether compound is formed. A "strongly bonded non-replaceable stable ligand" includes (i) a cyclopentadienyl ligand and (ii) a polymer-supported phosphine ligand.

In general, the present invention also includes a process for preparing epoxy-containing compounds without using halohydrins, by first making an allyl ether derivative compound of a phenolic compound or mixture of phenolic compounds as described in the present invention and subsequently epoxidizing the allyl derivative compound to an epoxy-containing compound. The process of epoxidizing an allyl derivative compound is taught, for example, in U.S. Patent 6,087,513. Herein, when reference is made to a "phenolic compound" or "phenolic compounds" it is meant to include a single phenolic compound or a combination of two or more phenolic compounds or mixtures of phenolic compounds.

U.S. Patent No. 5,578,740 discloses preferentially using an aqueous phase and a base for making allyl derivative compounds of active hydrogen-containing compounds. While the present invention process can also use an aqueous phase and a base in one embodiment of the present invention, it has been surprisingly found that the process for making allyl derivative compounds of phenolic compounds is more advantageous when an aqueous phase and a base (or a buffer) are not used and when the allylation reaction is carried out in the presence of a transition metal or rare earth metal catalyst, such as a palladium catalyst, having a strongly bonded ligand, such as a multidentate ligand or polymer bound ligand, for example, a polymer bound phosphine ligand; or in the presence of transition metal or rare earth metal catalysts, such as ruthenium or iridium catalysts, having at least one strongly bonded, non-replaceable ligand whose structure includes metal bonded ligands, such as cyclopentadienyl, and ligands with strongly coordinating heteroatoms, such as P, O, and N. Therefore, in one view, the present invention is an improved process over the process described in U.S. Patent No. 5,578,740 which uses a base or buffer in the allylation reaction and therefore produces a carboxylic acid salt by-product, such as sodium acetate, which can not be directly recycled to a process for making allyl carboxylates. Whereas, in the present invention, the allylation reaction produces a carboxylic acid by-product, such as acetic acid, which can be directly recycled to a process for making allyl carboxylates.

In general, the transition metal or rare earth metal of the catalyst used in the present invention includes, for example, ruthenium, rhenium, molybdenum, tungsten, rhodium, palladium, iridium, platinum, nickel, cobalt, iron, lanthnum, ytterbium, samarium, or any combination thereof. Preferably, ruthenium, iridium and palladium catalysts may be used in the present invention for allylation of active hydrogen-containing compounds, and especially for the allylation of aromatic compounds, such as phenols, when the specific ligands described previously are bound to the metal.

"Aryl allyl ether compounds" or "aryl allyl ethers" as used herein means (i) allyl ethers that contain an allyl ether moiety connected directly to an aromatic ring; or (ii) allyl ethers that contain an allyl ether moiety and an aromatic ring, but the allyl ether moiety is not connected directly to the aromatic ring, but instead the allyl ether moiety is linked to the aromatic ring via a linking group, such as a polyalkylene oxide group.

In one embodiment of the present invention, the aryl allyl ether or mixture of aryl allyl ethers prepared by the process of the present invention is represented by, but not limited to, the structures of the following Formulas I-V.

The following Formula I generically represents a preferred class of aryl allyl ethers of the present invention:

Formula I

(R¹)ₓ Ar(OR²)_{y}.

In Formula I, x is from 0 to 750, and y is from 1 to 150. When y is equal to or greater than two, then the aryl allyl ether is a multifunctional allyl ether.

In Formula I, Ar may be a moiety containing a mononuclear aromatic ring, such as for example, phenyl. Ar may also be a moiety containing multinuclear aromatic rings, such as for example, biphenyl, 2,2-diphenyl propane, bisphenylene oxide, tetrakis(1,1,2,2-phenyl)ethane, stilbene, phenol-formaldehyde novolac, cresol-formaldehyde novolac, phenol-dicyclopentadiene novolac and hyper-branched aromatic phenol dendrimers. Ar may also be a moiety containing multinuclear fused aromatic rings, such as for example, naphthalene, anthracene and naphthalene-formaldehyde novolac. Ar may also be a moiety containing multinuclear fused aromatic rings with one or more heteroatoms, such as for example, O, N, S, Si, B, P or any combination of these heteroatoms, such as for example, quinoxaline, thiophene and quinoline. Ar may also be a moiety containing mononuclear or multinuclear aromatic ring(s) fused with a cycloaliphatic ring(s), such as for example, indane, 1,2,3,4-tetrahydronaphthalene and fluorene. Ar may also be a moiety containing mononuclear or multinuclear aromatic ring(s) fused with a cycloaliphatic ring(s) containing one or more heteroatoms, such as for example, O, N, S, Si, B, P or any combination of these heteroatoms, such as for example, chroman, indoline and thioindane. Ar as described above in Formula I may also be partially or fully fluorinated.

In Formula I, Ar may also be a moiety containing aryl groups in which each aryl group may be connected to oligomeric (for example, polymers with less than 5000 molecular weight average) or high molecular weight (for example, greater than 5000 molecular weight average) organosiloxane units. In such case, the aryl groups may be attached directly to the Si atoms of the organosiloxane units, or the aryl groups may be indirectly attached to the Si atoms of the organosiloxane units via an organic aliphatic moiety, organic cycloaliphatic moiety, organic aromatic moiety, or any combination thereof. The organic aliphatic, cycloaliphatic, or aromatic moiety should contain no more than 20 carbon atoms. When the Ar moiety contains such oligomeric or high molecular weight organosiloxane units, then y is preferably from 2 to 150.

In Formula I, R¹ is a group substituted for a hydrogen atom on the aromatic ring(s) of the Ar moiety. R¹ may be a halogen atom, such as for example, bromo or chloro; or a hydrocarbon radical, such as for example, an alkyl group, a cycloaliphatic group or aromatic group. R¹ may be preferably an alkyl group having from 1 to 20 carbon atoms, such as for example, methyl, ethyl or propyl; a cycloaliphatic group having from 3 to 20 carbon atoms, such as for example, cyclopentyl or cyclohexyl; an aromatic group having from 6 to 20 carbon atoms, such as for example, phenyl or naphthyl; or any combination thereof. The hydrocarbon radicals above may also contain one or more heteroatoms, such as, for example, O, N, S, Si, B, P or any combination of these heteroatoms. An example of a hydrocarbon radical containing an O heteroatom is a methoxy group, an ethoxy group or a polyalkylene oxide group derived from ethylene oxide, propylene oxide, butylene oxide, cyclohexene oxide or any combination thereof. R¹ as described above in Formula I may be partially or fully fluorinated.

In Formula I, O is an oxygen atom substituted for a hydrogen atom on the aromatic ring(s) of the Ar moiety, and R² is a propenyl-containing moiety preferably selected from: 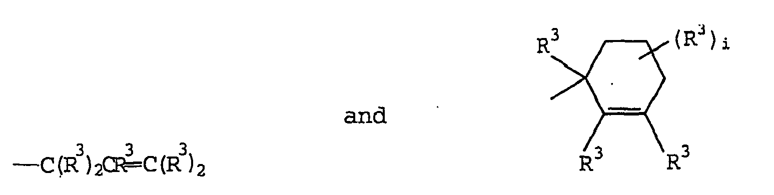 wherein R³ may be hydrogen; or R³ may be an alkyl group, a cycloaliphatic group or an aromatic group; and i is from 0 to 6. R³ may be an alkyl group having from 1 to 20 carbon atoms, such as for example, methyl, ethyl or propyl; a cycloaliphatic group having from 3 to 20 carbon atoms, such as for example, cyclopentyl or cyclohexyl; an aromatic group having from 6 to 20 carbon atoms, such as for example, phenyl or naphthyl; or any combination thereof. R³ may be partially or fully fluorinated. Each individual R³ may be the same group or may be a different group from each other.

In Formula I, R² may also be a monoalkylene oxide group or a polyalkylene oxide group derived from, for example, ethylene oxide, propylene oxide, butylene oxide or cyclohexene oxide; wherein each monoalkylene oxide group or each polyalkylene oxide group is terminated with a propenyl-containing moiety selected from: 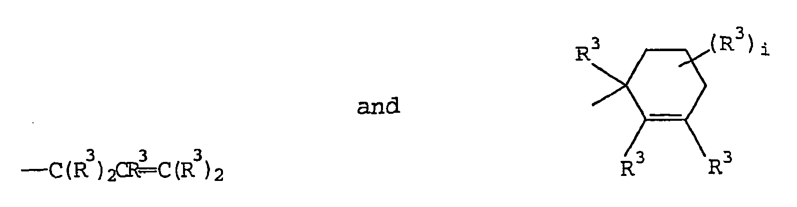 where R³ is as described above.

In one embodiment of the present invention, when R² in Formula I above is -CH₂CH=CH₂, the ether is an "allyl ether."

In another embodiment of the present invention, when R² is -CH₂C(CH₃)=CH₂ in Formula I above, the aryl ether is a "methallyl ether."

In yet another embodiment of the present invention, when R² in Formula I above is 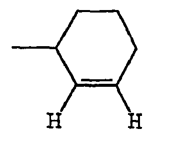 the aryl ether is a "cyclohexen-3-yl ether."

More specific and preferred examples of aryl allyl ethers useful in the present invention are represented by Formulas II-V separately or as mixtures of two or more aryl allyl ethers of Formulas II-V which follow.

Examples of mononuclear aryl allyl ethers of the present invention are represented by the following Formula II:

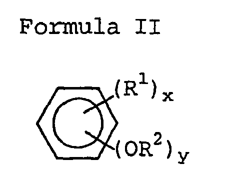

In Formula II, R¹, O and R² have the same meaning as described above with reference to Formula I. In Formula II, x is from 0 to 5 and y is from 1 to 4.

Other examples of aryl allyl ethers of the present invention are binuclear aryl allyl ethers which are represented by the following Formula III:

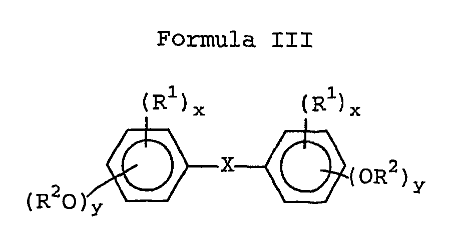

In Formula III, R¹, 0 and R² have the same meaning as described above with reference to Formula I. In Formula III, each x is from 0 to 4, and each x may be the same or different; and each y is from 1 to 4, and each y may be the same or different.

In Formula III, X may be nil; or X may be a heteroatom with or without substituents thereon to complete its necessary bonding valence; the heteroatom is preferably selected from O, N, S, Si, B, P, or any combination of two or more of the above heteroatoms. X may also be, for example, -C(O)-, -S(O₂)-, -C(O)NH-, or -P(O)Ar-. X may also be, for example, an organic aliphatic moiety, with or without heteroatoms, such as for example, oxydimethylene, methylene, 2,2-isopropylidene, isobutylene, or -CR³=CH-, where R³ is as defined with reference to Formula I above. X may also be, for example, a cycloaliphatic group, with or without heteroatoms, such as for example, a cycloaliphatic ring with greater than 3 carbon atoms; or an aromatic group, with or without heteroatoms; or any combination thereof, preferably with no more than 60 carbon atoms. X as described above in Formula III may be partially or fully fluorinated, such as for example, 2,2-perfluoroisopropylidene.

Other examples of aryl allyl ethers of the present invention are multinuclear aryl allyl ethers which are represented by the following Formula IV:

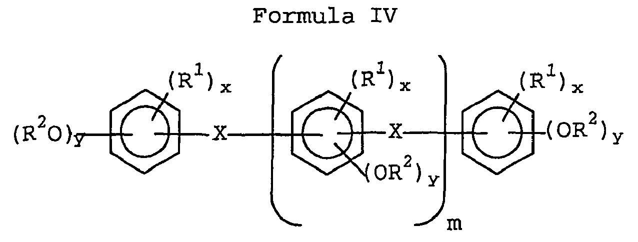

In Formula IV, R¹, O, R² and X have the same meaning as described above with reference to Formula III. In Formula IV, each x is from 0 to 4, and each x may be the same or different; and each y is from 1 to 4, and each y may be the same or different. In Formula IV, m is from 0.001 to 10.

Other examples of aryl allyl ethers of the present invention are multi-nuclear aryl allyl ethers which are represented by the following Formula V:

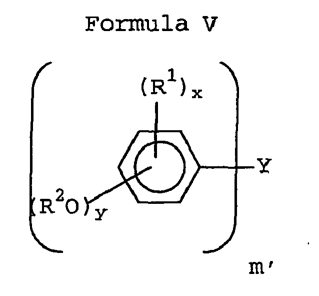

In Formula V, R¹, O and R² have the same meaning as described previously with reference to Formula I. In Formula V, each x is from 0 to 4, and each x may be the same or different, and each y is from 1 to 4, and each y may be the same or different.

In Formula V, Y may be an organic aliphatic moiety, with or without heteroatoms, such as, for example, O, N, S, Si, B, P or any combination of two or more of the above heteroatoms, wherein the aliphatic moiety has from 1 to 20 carbon atoms, such as for example, methine; a cycloaliphatic moiety, with or without heteroatoms, having from 3 to 20 carbon atoms, such as for example, cyclohexane tri-yl; an aromatic moiety, with or without heteroatoms, such as for example, benzenetriyl, naphthylenetriyl, fluorenetriyl; or any combination thereof, with no more than 20 carbon atoms. Y as described above in Formula V may be partially or fully fluorinated, such as for example, fluoromethine.

In Formula V, m' is generally 3 or 4. However, Y may also be an oligomeric (for example, less than 5000 molecular weight average) organosiloxane unit or high molecular weight (for example, greater than 5000 molecular weight average) organosiloxane unit. In which case, the aryl groups are attached to the Si atoms of the organosiloxane unit directly or through an organic aliphatic, cycloaliphatic, aromatic group, or any combination thereof, with no more than 20 carbon atoms. When Y is an oligomeric or high molecular weight organosiloxane unit, m' in Formula V is preferably from 1 to 150.

Even more particularly, the process of the present invention produces aryl diallyl ethers or aryl multifunctional allyl ethers. An example of an aryl diallyl ether of the present invention includes the diallyl ether of bisphenol A.

Typically, the aryl allyl ethers of the present invention are made from phenolic compounds or hydroxy-containing aromatic compounds or mixtures of phenolic compounds or mixtures of hydroxy-containing aromatic compounds.

The phenolic compound useful in the present invention contains at least one hydroxyl group; preferably contains on average more than one such hydroxyl group; and more preferably contains on average at least 1.8 such hydroxyl groups. The phenolic compound may contain up to 10 such hydroxyl groups. Examples of suitable phenolic compounds include mono-, di- or multi-functional aromatic hydroxyl-containing compounds or any combination of such compounds.

The hydroxy moiety of the hydroxy-containing aromatic compound may be directly attached to the aromatic ring of the hydroxy-containing aromatic compound, in which case it is a phenolic compound; or the hydroxy moiety may not be connected directly to the aromatic ring, but instead the hydroxy moiety may be linked to the aromatic ring via a linking group such as an alkylene group or polyalkylene oxide group.

Particularly suitable examples of the phenolic compounds used in the present invention include those which may be represented by any one of Formulas I through V, except that in the case of a phenolic compound, R² in Formulas I through V is H; or any combination of any two or more of such phenolic compounds represented by the above Formulas I through V, wherein R² is H.

More specific and preferred examples of phenolic compounds useful in the present invention include, for example, 1,2-, 1,3- and 1,4-dihydroxybenzene; 4-methyl-1,3-resorcinol; 1,4-, 1,5-, 2,5-, 1,7-, 1,6- and 2,6-dihydroxy naphthalene; 4,4'(3,3'5,5'-tetramethyl)bisphenol A; 4,4'(3,3'5,5'-tetramethyl-2,2',6,6'-tetrabromo)bisphenol A; 4,4'(3,3'5,'5'-tetramethyl)bisphenol F; 4,4'-dihydroxybiphenyl; 3,3',5,5'-tetramethyl-4,4'-dihydroxybiphenyl; 3,3',5,5'-tetramethyl-2,2',6,6'-tetrabromo-4,4'-dihydroxybiphenyl; bis(4-hydroxyphenyl)methane; bis(4-hydroxyphenyl)sulfone; 2,2-bis(3,5-dibromo-4-hydroxyphenyl)isopropylidene; 2,2-bis(4-hydroxyphenyl)isopropylidene; 4,4'-bisphenol K; 9,9-bis(4-hydroxyphenyl)fluorene; 4,4'-dihydroxy-α-methylstilbene; 1,3-bis(4-hydroxylphenyl)adamantane; phenol-formaldehyde novolac (functionality greater than 2); o-cresol-formaldehyde novolac (functionality greater than 2); phenol-dicyclopentadienyl novolac (functionality greater than 2); bisphenol-A-formaldehyde novolac (functionality >2) or other biphenol-formaldehyde novolac (functionality >2 ); naphthol-formaldehyde novolac (functionality ≥ 2); or naphthadiol-formaldehyde novolac (functionality ≥ 2); tris(4-hydroxyphenyl)methane; tris(3,5-dimethyl-4-hydroxyphenyl)methane; 1,1,2,2-tetrakis(4-hydroxyphenyl)ethane; and mixtures thereof.

The phenolic compound used in the present invention is preferably a bisphenol and is most preferably bisphenol A.

The allylating reagent useful in the present invention is preferably an allyl carboxylate or an allyl carbonate.

The allyl carboxylates useful in the present invention and from which the aryl allyl ether compounds can be prepared, include, for example, but are not limited to, those represented by the following Formula VI or any combination of any two or more such allyl carboxylates:

Formula VI

R⁴-C (=O)-O-R²
wherein R² is as defined with reference to Formula I; and R⁴ may be hydrogen, an alkyl group having from 1 to 4 carbon atoms, or an aromatic group having from 6 to 20 carbon atoms.

Preferably, the allyl carboxylate may be selected from allyl formate, allyl acetate, allyl propionate, allyl benzoate, and diallyl carboxylates such as diallyl oxalate, diallyl glutarate, diallyl succinate, or any combination thereof. Most preferred as the allyl carboxylate used in the present invention is allyl acetate.

Certain useful allyl carboxylates, such as allyl acetate, are commercially available. Others can be prepared, for example, by the oxidation of propylene in the presence of oxygen, a carboxylic acid (such as formic acid or propionic acid) and a palladium catalyst as described in U.S. Patent No. 3,970,713.

Suitable allyl carbonates which may be employed in the present invention include, for example, those represented by the following Formula VII or any combination of any two or more of such allyl carbonates:

Formula VII

R⁴-O-C (=O) -O-R²
wherein R² is as defined with reference to Formula I; R⁴ may be as defined with reference to Formula VI; or R⁴ may be the same as R².

Particularly suitable allyl carbonates which may be employed in the present invention include, for example, diallyl carbonate, dimethallyl carbonate, allyl methyl carbonate, allyl ethyl carbonate, allyl phenyl carbonate, or any combination of any two or more of such allyl carbonates.

The allyl carbonates may be prepared by methods well known in the art, for instance, by reacting dimethyl carbonate or phosgene with allyl alcohol. For example, allyl methyl carbonate, diallyl carbonate, or a mixture of allyl methyl carbonate and diallyl carbonate can be generated by reacting allyl alcohol with dimethyl carbonate in the presence of a catalytic amount of a base, such as, sodium hyroxide, sodium hydride or sodium methoxide and distilling out methanol formed during the process. Allyl methyl carbonate and/or diallyl carbonate or a mixture thereof prepared in this manner is suitable as the allylating reagent of the present invention.

The allylating reagent of the present invention is employed in amounts such that the equivalent ratio of the allylating reagent to the hydroxyl group of the phenolic compound is from 0.1 to 500 equivalents of allylating reagent per one equivalent of hydroxyl group, preferably from 0.5 to 50 equivalents of allylating reagent per one equivalent of hydroxyl group, and more preferably from 1 to 20 equivalents of allylating reagent per one equivalent of hydroxyl group.

At ratios significantly above one equivalent of allylating reagent per one equivalent of hydroxyl group, the excess allylating reagent is also being employed as a solvent in addition to its use as a reactant. At ratios below about one equivalent of allylating reagent per one equivalent of hydroxyl group, conversion to the desired product may be very low and the excess phenolic reactant may have to be recovered and recycled in the process.

The catalyst useful in the present invention in the allylation reaction process is a transition metal or rare earth metal catalyst complexed with a strongly bonded, non-replaceable stable ligand. The ligand of the transition metal or rare earth metal catalyst complex may be (i) an olefinic-containing ligand or an aromatic-containing ligand; or (ii) a polymeric ligand or a heteroatom-containing multidentate ligand.

Suitable catalysts used in the present invention for allylation of the phenolic compounds are represented by the following general structure Formula (A):

Formula (A)

[M⁺ⁿₛOₜ(H)ᵥ(L¹)ᵥ(L²)ᵥ(L³)ᵥ(L⁴)ᵥ(L⁵)ᵥ(L⁶)ᵥ...(L^{p})ᵥ]_{w}
wherein M is a transition metal or rare earth metal; n is the oxidation state of metal M, and n is from 0 to 8; and s is an integer from 1 to 5, and preferably from 1 to 3.

O is oxygen such that in the above catalyst Formula (A) the oxygen bonds to metal to form a metal oxo moiety having the following structure:
M=O;
or O is oxygen such that in the above catalyst Formula (A) the oxygen bonds to metal M and to H, L or to a second M to form a metal µ-oxo moiety having one of the following structures:
   M-O-H, M-O-M, or M-O-L;
   and t is an integer from 0 to 3.

In the above catalyst complex Formula (A), H is a hydrogen atom which is bonded to the metal atom M or is bonded to the oxygen of a metal-µ-oxo moiety to form one of the following structures:
M-H or M-O-H.

Each one of L¹, L², L³, L⁴, L⁵, L⁶...L^{p} in the above general catalyst complex Formula (A), represents a ligand which may be the same ligand or a different ligand up to a maximum number of p ligands bound to the metal M; v is an integer from 0 to 10; and v may be the same for H and any of L¹, L², L³, L⁴, L⁵, L⁶...L^{p} or v may be different for H and any of L¹, L², L³, L⁴, L⁵, L⁶...L^{p}; and w is an integer from 1 to 500. Overall Oₜ(H)ᵥ(L¹)ᵥ(L²)ᵥ(L³)ᵥ(L⁴)ᵥ(L⁵)ᵥ(L⁶)ᵥ...(L^{p})ᵥ satisfies the bond nature of the transition metal or rare earth metal; and the sum of s, t, v and w satisfies the bond nature of the transition metal or rare earth metal. For a d orbital (d°) element metal, the total number of electrons in the d orbital is preferred to be less than or equal to 18.

In one embodiment of the present invention, the transition metal or rare earth metal portion, M, of the transition metal or rare earth metal catalyst Formula (A) used in the present invention is a transition metal or rare earth metal selected from the group comprising ruthenium, rhenium, molybdenum, tungsten, rhodium, palladium, iridium, platinum, nickel, cobalt, iron, lanthanum, ytterbium, samarium or any combination thereof; more preferably, M is ruthenium, iridium, or platinum.

In another embodiment of the present invention, when s in Formula (A) above is greater than 1, when w is greater than 1, or when s is equal to 1 and w is greater than 1, M may be the same transition metal or rare earth metal, preferably ruthenium, iridium, or platinum, or the combination of ruthenium, iridium, or platinum with other transition metals or rare earth metals, such as, ruthenium, rhenium, molybdenum, tungsten, rhodium, palladium, iridium, platinum, nickel, cobalt, iron, lanthanum, ytterbium and samarium provided that at least one of such metal atom is ruthenium, iridium, or platinum.

Another embodiment of the transition metal or rare earth metal catalyst of Formula (A) useful as catalyst in the present invention may include, for example, bi-metal complexes, poly-metal complexes, bi-metal oxo complexes, poly-metal oxo complexes, and metal clusters. In such embodiment, the metal atoms may be bound to one another through metal-metal bonds, but are not required to be bound to one another. Alternatively, the metal atoms may be bound to each other through an oxygen atom such as in a metal µ-oxo moiety. The metal atoms may also be bound to each other through bidentate or multidentate ligands, which include oxygen-containing ligands, nitrogen-containing ligands, phosphorous-containing ligands, organosilyl-containing ligands, and organosilyloxy-containing ligands. "Multidentate ligand" means a ligand that contains three or more heteroatoms such as O, N, S, Si, B, P.

In another embodiment of the present invention, in the ligand portion L¹, L², L³, L⁴, L⁵, L⁶...L^{p} of the transition metal or rare earth metal catalyst of Formula (A) used in the present invention, at least one or more of the ligands L¹, L², L³, L⁴, L⁵, L⁶...L^{p} is a "strongly bonded, non-replaceable stable ligand or complexing agent." The "strongly bonded, non-replaceable stable ligand or complexing agent" portion of the transition metal or rare earth metal catalyst of Formula (A) used in the present invention is preferably (i) an olefinic ligand or an aromatic-containing ligand; or (ii) a polymeric ligand or a heteroatom-containing multidentate ligand.

In yet another embodiment of the present invention, it has been found that the at least one or more of the "strongly bonded, non-replaceable stable ligand or complexing agent" L¹, L², L³, L⁴, L⁵, L⁶...L^{p} portion of the transition metal or rare earth metal catalyst of Formula (A) used in the present invention is an unsaturated aliphatic or an aromatic moiety-containing ligand. The unsaturated aliphatic or aromatic moiety-containing ligands include neutral, monodentate ligands, bidentate ligands, and multidentate ligands. As illustrations of other embodiments of the present invention, the following Formulas (B) through (G) are described and represent partial metal complex structures showing the various ways in which ligands having olefinic or aromatic moieties can be complexed with the transition metal or rare earth metal M of the transition metal or rare earth metal catalysts of Formula (A).

Formula (B) as follows is a partial structure of the transition metal or rare earth metal catalyst useful in the allylation process of the present invention:

Formula (B)

R⁵-M

In Formula (B), M is a transition metal or rare earth metal and R⁵ may be a monodentate olefinic or an aromatic-containing ligand, such as for example, cyclopentadienyl (Cp), pentamethylcyclopentadienyl (Cp*), indenyl (In), 9-fluorenyl(Fl), cyclooctadienyl (COD) or butyldienyl ligands. The monodentate Cp-, Cp*-, In-, Fl- and COD-containing ligands are described by S. Hitchcock in Organometallics, 14, pp.3732-3740, (1995).

The metal complexes having one monodentate olefinic or aromatic-containing ligand, or the substituted analogue thereof, are a very well-known class of compounds known as "half-shell" metal complexes.

"Half-shell" transition metal or rare earth metal catalysts useful as allylation catalysts in the present invention which incorporate the partial Formula (B) into Formula (A) include, but are not limited to for example, Cp*RuCl(PPh₃)₂; Cp*RuCl(dppe); Cp*RuCl(COD); CpRuCl(PPh₃)₂; CpRuCl (dppe); CpRuCl (COD); CpRu⁺(PPh₃)₂PF6⁻; CpRu⁺(PPh₃)₂CF₃SO₃⁻; wherein "dppe" is 1,2-bis(diphenylphosphino)ethane.

Formula (C) set forth below is a partial structure of the transition metal or rare earth metal catalyst useful in the allylation process of the present invention:

Formula (C)

R⁵-M-R⁵

In Formula (C), M is a transition metal or rare earth metal, and R⁵ is as defined above with reference to Formula (B), wherein each R⁵ may be the same or different.

The above partial metal complex Formula (C) having two monodentate olefinic or aromatic-containing ligands or the substituted analogues thereof represents the partial structure of a very well-known class of compounds known as "sandwich" metal complexes.

"Sandwich" transition metal or rare earth metal catalysts of Formula (A), which incorporate the partial Formula (C) into Formula (A), useful as allylation catalysts in the present invention include, but are not limited to for example, (Cp)₂RuCl; (Cp)₂RuBr; (Cp*)₂RuCl; (Cp*)₂RuBr; (In)₂RuCl.

Formula (D) set forth below is a partial structure of the transition metal or rare earth metal catalyst useful in the allylation process of the present invention:

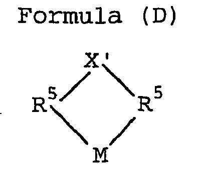

In Formula (D), M is a transition metal or rare earth metal; R⁵ is as defined above with reference to Formula (B); and X' is a moiety bridging or linking the R⁵ groups together such that the R⁵-X'-R⁵ structure becomes a bidentate ligand, wherein R⁵ may be the same group or a different group. When the R⁵ groups in Formula (D) are different groups, R⁵ may be a part of a ligand having O, P, N, S, or Si atoms. X' may be, for example, methylene, ethylene, isopropylidene, binaphthyldimethylene, dimethylsilylene, bis(dimethysilyl)oxy.

The above partial metal complex Formula (D) having two monodentate olefinic or aromatic-containing ligands or the substituted analogues thereof connected by a bridging or linking moiety represents a partial structure of a very well-known class of compounds known as "ansa" metal complexes.

"Ansa" bidentate strongly bonding ligands of Formula (A) useful as allylation catalysts in the present invention include, but are not limited to for example, bis(4-t-butyl-2-methylcyclopentadienyl)dimethylsilane; bis(cyclopentadienyl)dimethylsilane; bis(9-fluorenyl)dimethylsilane; bis(1-indenyl)dimethylsilane; bis(2-methyl-1-indenyl)dimethylsilane; cyclopentadienyl-(9-fluorenyl)diphenylmethane; cyclopentadienyl-(9-fluorenyl)dimethylsilane; 1,2-bis(1-indenyl)ethane; 1,2-bis(2'-methyl-1-indenyl)ethane; 1,2-bis(4',5',6',7'-tetrahydro-1-indenyl)ethane; 2,2-(cyclopentadienyl)-(9-fluorenyl)propane; bis-(1-benzoindenyl)dimethylsilane.

Formulas (E), (F), and (G) set forth below represent partial structures of three additional "ansa"-type transition metal or rare earth metal complexes useful in the allylation process of the present invention wherein one or more of the transition metal or rare earth metal to ligand bonds is formed through an ancillary group A:

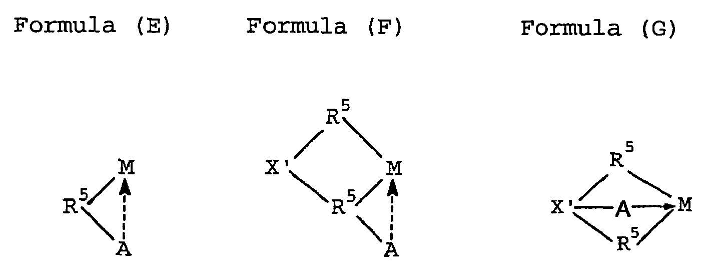

In Formulas (E), (F), and (G), M is a transition metal or rare earth metal; R⁵ and X' are as defined above with reference to Formula (B); and A is an ancillary group attached to the R⁵ or X' group, wherein A may be an aliphatic, cycloaliphatic, aromatic, or combination thereof moiety having one or more heteroatoms, such as O, N, S, Si, B or P, capable of forming one or more coordination bonds with the transition metal or rare earth metal M. When A has more than one heteroatom, the heteroatoms may be the same or different.

An A moiety includes, for example, but is not limited to, an alkyl, cycloalkyl, aromatic amine or diamine or any combination thereof; an alkyl, cycloalkyl, aromatic alcohol or diol or any combination thereof; an alkyl, cycloalkyl, aromatic ether or diether or any combination thereof; an alkyl, cycloalkyl, aromatic phosphine or diphosphine or any combination thereof; an alkyl, cycloalkyl, aromatic silane or disilane or any combination thereof, with or without fluorine atoms.

Additionally, an A moiety having two heteroatoms includes, for example, but is not limited to, alkyl, cycloalkyl, aromatic, or any combination thereof amino alcohol, amino ether, amino phosphine, amino silane, ether alcohol, phosphine alcohol, silane alcohol, ether phosphine, ether silane, phosphine silane, with or without fluorine atoms. When an ancillary A moiety has more than one heteroatom, the R⁵-A ancillary group may be a bidentate or multidentate ligand capable of forming two or more coordination bonds with the transition metal or rare earth metal.

"Ansa"-type ligands, which have an ancillary group A and which are strongly bonding ligands of Formula (A) useful as allylation catalysts in the present invention include, for example but are not limited to, bis(Cp*)(3-methoxy-1-propyl)methylsilane; bis(Cp*)(3-methoxy-1-pentyl)methylsilane; dimethyl (dimethylaminoethylcyclopentadienyl) (Cp*)silane; trimethyl(2-methoxyethylcyclopentadienyl)silane; trimethyl(2-isobornyloxyethylcyclopentadienyl)silane; trimethyl(2-menthylethyloxycyclopentadienyl)silane; trimethyl(2-fenchyloxyethylcyclopentadienyl)silane; N,N-dimethyl-2-aminoethylcyclopentadiene; N,N-dimethyl-3-aminopropylcyclopentadiene; (Cp)dimethyl(diphenylphosphinomethyl)silane.

Examples of "ansa"-type transition metal or rare earth metal catalysts of Formula (A) of the present invention include, but are not limited to, [Cp-C(=O)-O-(CH₂)₂-PPh₂]Ru⁺(MeCN)₂ PF6⁻ as described by S. Takahashi in Chem. Commun., pp. 521-522, (2001); and Cp*Cl [(C₆F₅)₂PCH₂CH₂P(C₆F₅)₂] Ru⁺BF₄⁻ as described by C. Suander in Dalton Communication, pp. 512-514, (2001).

In another embodiment of the present invention, the unsaturated aliphatic or aromatic ligand may be bonded to an organic, an inorganic, or a hybrid organic-inorganic polymeric backbone. Examples of such unsaturated aliphatic or aromatic moiety-containing polymeric ligands are described by C. H. Brubaker, Jr., et. al, in J. of Organometallic Chemistry, 214, 325-337, (1981) and by P. Leeuwen in Angew. Chem. Int. Ed., 40, pp. 1828-1849, (2001).

In another preferred embodiment of the present invention, it has been found that the at least one or more "strongly bonded, non-replaceable stable ligand or complexing agent", L¹, L², L³, L⁴, L⁵, L⁶...L^{p} portion of the transition metal or rare earth metal catalyst of Formula (A) used in the present invention may be a O-, N-, S-, Si-, B-, or P-containing polymeric ligand or a multidentate phosphorous ligand.

When the strongly bonded, non-replaceable or essentially non-replaceable, stable ligand useful for the transition metal or rare earth metal catalyst of Formula (A) in the present invention is a polymeric ligand, the polymeric ligand can consist of an organic polymeric backbone, an inorganic polymeric backbone, or a hybrid organic-inorganic polymeric backbone. Because of their polymeric nature these strongly bonded, non-replaceable or essentially non-replaceable, stable polymeric ligands are multidentate ligands. The polymeric ligands may contain heteroatoms such as O, N, S, Si, B, P or any combination of two or more of these heteroatoms. The polymeric ligands may also contain one or more asymmetric or chiral centers.

Examples of heteroatom-containing polymeric ligands having organic polymeric backbones include, but are not limited to for example, styrene-co-divinyl benzene, vinyl pyridine-co-divinyl benzene and aromatic polyimide crosslinked polymers having substituted phosphorous moieties bound to the backbone. Such phosphorous-containing polymeric ligands are well-known in the art and are described, for example, in Polymer-supported Reactions in Organic Synthesis, edited by P. Hodge and D. C. Sherrington, John Wiley & Sons, 1980 ; by U. Yasuhiro in Tetrahedron, 55, pp. 14341-14352, (1999) and in J. Org. Chem., 64, pp. 3384-3388, (1999); and by, S. Ley in J. Chem. Soc., Perkin Trans. I, pp. 3815-4196, (2000).

Examples of heteroatom-containing polymeric ligands having hybrid organic-inorganic ligands, include, but are not limited to, for example, polymeric ligands having organo-sol gel backbones. Polymeric ligands having organo-sol gel backbones include, for example, the polymeric organophosphine ligands having organosiloxane backbones described by E. Lindner in J. Organometallic Chem., 628(2), pp. 151-154, (2001), in Angew. Chem. Int. Ed., 38, pp. 2154-2157, (1999), and in J. Non-Crysataline Solids, 225, pp. 208-216 (1999); and in U.S. Patent No. 5,620,938. Polymeric ligands having organo-sol gel backbones also include, for example, polymeric organonitrogen ligands having organosiloxane backbones and containing pyridinyl moieties as described by S. Yu in Angew. Chem. Int. Ed., 40, pp. 437-440, (2001). Polymeric ligands having organo-sol gel backbones may also include, for example, polymeric organonitrogen ligands or polymeric organophosphine ligands having dendrimeric organosiloxane backbones such as described by P. Leeuwen in Angew. Chem. Int. Ed., 40, pp. 1828-1849, (2001).

When the ligands used in the present invention are polymeric ligands, the transition metal or rare earth metal of the transition metal or rare earth metal catalyst is anchored onto the polymeric backbone.

In one embodiment of the present invention, the strongly bonded, non-replaceable or essentially non-replaceable, stable ligands useful for the transition metal or rare earth metal catalyst in the present invention include, for example, the multidentate phosphorous-containing ligands. The multidentate phosphorous-containing ligands may also contain heteroatoms such as O, N, S, Si, B or any combination of two or more of these heteroatoms. The multidentate phosphorous-containing ligands may also contain one or more asymmetric or chiral centers.

The strongly bonded, non-replaceable, stable multidentate phosphorous-containing ligands, which may be useful in the present invention include, but are not limited to, the tri-, tetra, penta- and greater-phosphorous-containing ligands, such as cis-cis-cis-1,2,3,4-tetrakis(diphenylphosphinomethyl)cyclopentane as described by M. Santelli, et. al, in J. Org. Chem., 66(5), pp. 1633-1637,(2001); and as described in U.S. Patent No. 6,087,513.

When the ligand used in the present invention is a polymeric or a multidentate ligand, it may be bonded to the same metal atom or to two or more different metal atoms.

While it is desired to have at least one or more strongly bonded, non-replaceable or essentially non-replaceable, stable ligands L¹, L², L³, L⁴, L⁵, L⁶...L^{p} attached to the transition metal or rare earth metal of the transition metal or rare earth metal catalyst of Formula (A), it is also possible that the transition metal or rare earth metal of the catalyst contains at least one or more vacant coordination sites or at least one or more labile ligands L¹, L², L³, L⁴, L⁵, L⁶...L^{p} to allow for the insertion of the allylating agent onto the transition metal or rare earth metal in order to activate the allylic moiety to carry on the allylation process. The at least one or more labile ligands L¹, L², L³, L⁴, L⁵, L⁶...L^{p} of the transition metal or rare earth metal catalyst of Formula (A) are also called "hemilabile" hybrid ligands as described and illustrated by P. Braunstein, et al., in Angew. Chem. International Edit., 40(4), pp. 680-699, (2001); and by C. Bessel, et al., in Chem. Rev., 101, pp. 1031-1066, (2001).

The steric nature and electronic nature of both the strongly bonded, non-replaceable or essentially non-replaceable, stable ligands L¹, L², L³, L⁴, L⁵, L⁶...L^{p} and the labile ligands or so called "hemilabile" hybrid ligands L¹, L², L³, L⁴, L⁵, L⁶... L^{p} may be varied to change the properties of the transition metal or rare earth metal atom(s) of the catalyst and hence the catalytic properties of the complexed metal salt or the transition metal or rare earth metal catalyst complex useful in the present invention when such complexed metal salt or transition metal or rare earth metal catalyst complex is combined with an allylating agent.

Varying the steric nature and the electronic nature of ligands may be done according to the teachings by R. Crabtree in The Organometallic Chemistry of the Transition Metals, Second Edit., John Wiley & Sons, N.Y. (1994); by A. Yamamoto in Organotransition Metal Chemistry, John Wiley & Sons, N.Y. (1986); and by F. Cotton and G. Wilkinson in Advanced Inorganic Chemistry, Fourth Edition, John Wiley & Sons, N.Y. (1980).

The ligands L¹, L², L³, L⁴, L⁵, L⁶...L^{p} useful in the present invention, as represented by Formula (A), may also include both strongly bonded and labile ligands, such ligands include, but are not limited to, the following representative ligands: (i) oxygen-containing ligands, (ii) phosphorous-containing ligands, (iii) nitrogen-containing ligands, (iv) silicon-containing ligands, and (v) anionic ligands.
(i) Monodentate, bidentate or polydentate oxygen-containing ligands are ligands L useful for the preparation of transition metal or rare earth metal catalysts of Formula (A) of the present invention. Monodentate, bidentate or polydentate oxygen-containing ligands useful in the present invention include, but are not limited to, for example, aliphatic and cycloaliphatic alcohols and diols; phenolates; carboxylic acids; ketones; phenoxy imine compounds or the combination of these oxygen-containing ligands. Partially or fully fluorinated monodentate or polydentate oxygen-containing ligands are also included herein as examples useful in the present invention.
   Examples of oxygen-containing ligands useful in the present invention are described in U.S. Patent No. 6,087,513, including for example, propane-1,2-diol; cis- and trans-cyclohexane-1,2-diol; and 1,1'-binaphthyl-2,2'-diol.
(ii) Monodentate or bidentate phosphorous-containing ligands are ligands L useful for the preparation of transition metal or rare earth metal catalysts of Formula (A) of the present invention. Monodentate or bidentate phosphorous-containing ligands useful in the present invention include, for example, aliphatic, cycloaliphatic or aromatic organophosphinyl ligands.
   Examples of labile, non-stable monodentate phosphorous-containing ligands useful in the present invention include, but are not limited to, for example, trimethylphosphine; triisopropylphosphine; triphenylphosphine; tricyclohexylphosphine or any combination of these phosphorous-containing ligands.
   Strongly bonded, neutral or basic, bidentate ligands containing aliphatic, cycloaliphatic, or aromatic organophosphinyl ligands useful in the present invention include bidentate ligands, such as for example, but not limited to, 1,2-bis(diphenylphosphino)ethane; 2-(diphenylphosphinyl)cyclohexanol; 2,2'-bis(diphenylphosphino)-1,1'-binaphthyl; 1,1'-bis(diphenylphosphino)ferrocene; 1,2-bis(2,5-dimethylphospholano)ethane; endo,endo-3-(diphenylphosphoryl)-2-hydroxybornane.
   Partially or fully fluorinated monodentate or didentate phosphorous-containing ligands are also included herein as examples useful in the present invention.
(iii) Nitrogen-containing ligands such as monodentate or polydentate amino compounds, heterocyclic ring compounds, biheterocyclic ring compounds or fused heterocyclic ring compounds are ligands L useful for the preparation of transition metal or rare earth metal catalysts of Formula (A) of the present invention. The nitrogen-containing ligands may contain heteroatoms other than nitrogen such as O, S, Si, B, P, or any combination of these heteroatoms. Partially or fully fluorinated monodentate or polydentate nitrogen-containing ligands are also included herein as examples useful in the present invention.
   Included herein, but not intended to be a complete listing of examples of the nitrogen-containing ligands useful in the present invention, are multiaza, acyclic, cyclic, and macrocyclic amines, such as for example, tris[2-(N-trimethylsilyl)aminoethyl]amine; 1,3,5-trimethyl-1,3,5-triazacyclohexane; cyclam-(1,4,8,11-tetraazacyclodecane); 1,2-cyclohexanediamine-N,N'-bis(3,3-di-t-butylsalicylidene); ethylenebis(salicylimine); 2,6-bis(imino)pyridyl ligands.
   The nitrogen-containing monodentate and multidentate monoheterocyclic and multiheterocyclic ligands useful in the present invention include, for example, but are not limited to, monoheterocyclic pyridine ligands, such as, (2'-pyridyl)propan-2-ol; 9-(2-pyridyl)-9-fluorenol; bis[4-(diethylamino)phenyl]-2-pyridylmethanol; 6-(2-hydroxyphenyl)pyridine-2-carboxylic acid.
   Monodentate and multidentate monoheterocyclic and multiheterocyclic ligands useful in the present invention also include, for example, but are not limited to, multiheterocyclic pyridine ligands such as tris(2'-pyridylaminodimethyl silyl)methane; tris(2'-(4,6-dimethylpyridyl)aminodimethyl silyl)methane; 2,2'-bipyridine.
   Monodentate and multidentate monoheterocyclic and multiheterocyclic ligands useful in the present invention include, for example, but not limited to, multiheterocyclic pyridine-oxazolin, pyridine-pyrazol, and pyrazine-pyrazol ligands, such as, 2,6-bis[4-methyloxazolin-2-yl]pyridine; 4-hydroxymethyl-5-phenyl-2-(2-pyridinyl)4,5-dihydro[2,1-d]oxazole; 2-(5-methylpyrazol-3-yl)pyridine; 2-(4-chloropyrazol-3-yl)pyridine; 2-(5-methyl-1-octylpyrazol-3-yl)pyrazine.
   Nitrogen-containing ligands useful in the present invention include biheterocyclic quinoline and biheterocyclic oxazoline ligands, such as for example, but are not limited to, 2-methyl-8-hydroxyquinoline; 2,2'-methylenebis(oxazoline).
   Nitrogen-containing ligands useful in the present invention also include nitrogen-containing heterocyclic ligands that contain boron, such as for example, but not limited to, hydrotris(3,5-dimethyl-1-pyrazolyl)borate.
   Partially or fully fluorinated monodentate or polydentate nitrogen-containing ligands are also included herein as examples useful in the present invention.
(iv) Silicon-containing ligands such as monodentate, didentate or polydentate silyl or silyloxy ligands L are useful for the preparation of transition metal or rare earth metal catalysts of Formula (A) of the present invention.
   The incorporation of silicon-based ligand L, within the transition metal or rare earth metal complexes of Formula (A) of the present invention is important not only because it may provide the chemical functionality for bonding of the transition metal or rare earth metal complexes to solid supports, but also the silicon-based ligands may impart advantageous solubility and hydrolytic stability properties to the catalyst.
   Silicon-containing ligands can be generally divided into two groups: (1) organosilyl-containing ligands and (2) organosilyloxy-containing ligands.
   In the organosilyl-containing ligands, the silicon atom is directly bonded to the transition metal or rare earth metal atom. An example of a monodentate organosilyl-containing ligand is -Si(R³)₃ and an example of bidentate or multidentate organosilyl-containing ligand is -Si(R³)2[-O-Si(R³)₂]m"-O-Si(R³)₂- where R³ is the same as described above and m" is from 0 to 10. The method used to make such organosilyl-containing ligands wherein the silicon atom is directly linked to the transition metal is taught, for example, by W. Malisch in Inorg. Chem., 34, 23, pp. 5701-5702, (1995).
   Organosilyl-containing ligands may also be introduced into a transition metal or rare earth metal complex by indirectly attaching the organosilyl-containing moiety to the transition metal or rare earth metal atom through a monodentate ligand, such as -L-Si(R³)₃ wherein L is a ligand as defined above. An example of such a monodentate organosilyl-containing ligand is γ-aminopropyltrimethoxysilane, wherein an amido moiety is attached to one terminus and an organosilyl moiety is attached to the other terminus of the ligand such as described in U.S. Patent No. 5,620,938. organosilyloxy-containing ligands, may be monodentate-ligands such as -O-Si(R³)₃ or multidentate ligands such as -O-Si(R³)₂[-O-Si (R³)₂]_{m"}-O-Si (R³)₂-O-, wherein the silicon atom is indirectly bonded to the transition metal or rare earth metal through an oxygen atom, where R³ and m" are as defined above.
   Organosilyloxy-containing ligands may also be introduced into a transition metal or rare earth metal complex by indirectly attaching the organosilyoxyl-containing moiety to the transition metal or rare earth metal atom through a monodentate ligand, such as -L-O-Si(R³)₃ wherein L is as defined above. One such method to introduce an organosilyloxy-containing ligand into a transition metal complex is taught, for example, by W. Malisch in Inorg. Chem., 34, 23, pp.5701-5702 (1995).
   When a transition metal or rare earth metal complex with mono-, di- or trifunctional organosilyl groups is condensed with mono-, di-, tri- or tetramethoxy or ethoxy silane, a three-dimensional organic-inorganic hybrid material is formed. Depending on the degree of crosslinking, such hybrid organic-inorganic material is soluble or partially soluble in organic solvents. The process making soluble or partially organic-soluble hybrid organic-inorganic materials useful in the present invention is described by K. J. Shea, et al., in Chem. Rev., 95, pp. 1431-1442 (1995).
   When transition metals or rare earth metals with ligands are bonded to a silasesquioxane, a three-dimensional, organic soluble metallosiloxane is formed. The organic soluble, three-dimensional, transition metal or rare earth metal silasesquaoxane can be anchored or bonded onto a silicate-based, solid support to form a heterogeneous catalyst. When the transition metal or rare earth metal is connected three-dimensionally through M-O-Si bonds, it is very tightly anchored onto the solid support.
   In the organosilyl-containing ligands, the silicon atom may also be directly bonded to the transition metal or rare earth metal atom. The silicon atom of organosily-containing ligands may also be bound to aliphatic, cycloaliphatic or aromatic segments or radicals. The silicon atom of organosily-containing ligands may also be bound to an organophosphinyl group. The silicon atom of organosily-containing ligands may also be bound to polymeric backbones or to oligomeric alkalene oxide chain(s), such as those described by E. Lindner, in J. Organometallic Chem., 628(2), pp. 151-154, (2001) and in J. Non-Crysatlline Solids, 225, pp. 208-216, (1999).
(v) The ligands L¹, L², L³, L⁴, L⁵, L⁶...L^{p} in Formula (A) may also be selected from anionic groups. When n of Formula (A) is greater than zero, then anionic or negatively charged ligands, such as, chloride, bromide, acetate, nitrate, sulfate, tosylate, triflate, and hexaflurophosphonate; or the combination of any two or more of these anionic ligands; may be useful as ligands in the transition metal or rare earth metal catalysts of the present invention.

The above described ligands L¹, L², L³, L⁴, L⁵, L⁶...L^{p} portion of the transition metal or rare earth metal catalyst of Formula (A) are meant to be representative of and not all inclusive of the ligands or complexing agents useful in the present invention. A more detailed definition of the ligands useful in the present invention can be found in U.S. Patent No. 6,087,513.

Ligands or complexing agents are preferably employed to stabilize and to enhance the activity of the transition metal or rare earth metal catalyst of Formula (A). The transition metal or rare earth metal of the catalyst of Formula (A) may be complexed with the complexing agent or ligand prior to addition to the allylation reaction mixture, in which instance the transition metal or rare earth metal catalyst is often isolated having a stoichiometric ratio of ligand(s) to transition metal or rare earth metal; or the catalyst complex may be formed in situ by adding the transition metal or rare earth metal and the ligand or complexing agent separately to the reaction mixture, in which instance the molar ratio of ligand or complexing agent to transition metal or rare earth metal may vary over a wide range. The complexing agents or ligands may be employed in amounts of from 0.5 to 20 equivalents of complexing atom or moiety per one equivalent of transition metal or rare earth metal catalyst, preferably from 0.75 to 10 equivalents of complexing atom or moiety per one equivalent of transition metal or rare earth metal catalyst, more preferably from 1 to 5 equivalents of complexing atom or moiety per one equivalent of transition metal or rare earth metal catalyst.

The allylation catalyst of Formula (A) of the present invention may be employed as a homogenous or a heterogeneous catalyst.

In one embodiment of the present invention, the allyation catalyst of Formula (A) is a homogeneous catalyst. In such homogeneous catalysts, the transition metal or rare earth metal catalyst is soluble in the allylation reaction mixture.

When the catalyst is employed as a homogeneous catalyst, the reaction may be conducted in a batch or continuous mode.

In another embodiment of the present invention, the transition metal or rare earth metal allylation catalyst of Formula (A) is a heterogeneous catalyst. By heterogeneous catalyst it is meant that the transition metal or rare earth metal catalyst is either (i) insoluble in the reaction mixture or (ii) placed onto a solid support material which is insoluble in the allylation reaction mixture.

Heterogeneous catalysts may be formed by any of three separate processes: (i) by supporting the transition metal or rare earth metal on a support material through simple adsorption of the metal catalyst on the support material via deposition of the catalyst on the support material; (ii) by supporting the transition metal or rare earth metal on a support material through anchoring or tethering of the transition metal or rare earth metal catalyst of Formula (A) to a the support material via bond formation between a reactive site on the transition metal or rare earth metal catalyst of Formula (A) and the support material; or (iii) by forming the transition metal or rare earth metal catalyst of Formula (A) by reacting a transition metal or rare earth metal complex with a solid polymeric ligand. The heterogeneous catalysts may be made by any of several procedures well-known to those skilled in the art.

The heterogeneous catalyst of Formula (A) of the present invention may be formed via process (i) by direct deposition of the transition metal or rare earth metal catalyst on the solid support, wherein the metal catalyst is simply absorbed onto the solid support, such as described for example by H.-U. Blaser, et. al, in J. of Molecular Catalysis A: Chemical, 173, pp. 3-18, (2001); and by D. Stirling, et. al, in J. of Molecular Catalysis A: Chemical, 172, pp. 207-218, (2001). The heterogeneous catalyst made by the process of depositing the transition metal or rare earth metal catalyst on the solid support may be utilized directly or the heterogeneous catalyst may be subsequently treated by any of a number of methods, such as calcination, well-known to those skilled in the art.

The heterogeneous catalyst of Formula (A) of the present invention may be formed via process (ii) by anchoring or tethering the transition metal or rare earth metal catalyst to a solid support. By anchoring or tethering the transition metal or rare earth metal catalyst to a solid support it is meant that a stable, ionic, coordination or covalent chemical bond is formed between at least one of the L¹, L², L³, L⁴, L⁵, L⁶...L^{p} ligands portion of the transition metal or rare earth metal catalyst of Formula (A) and the solid support, therefore rendering the transition metal or rare earth metal catalyst into the heterogeneous state. Such a process (ii) for formation of a heterogeneous catalyst is described, for instance, by B. Chaudret, et. al, in J. Am. Chem. Soc., 123, pp.7584-7593, (2001).

In the processes (i) and (ii) for making a heterogeneous catalyst of the present invention, the solid support may include, for example, but is not limited to, styrene-co-divinyl benzene or vinyl pyridine-co-divinyl benzene crosslinked polymers or ion exchange resins, aromatic polyimides, organosol gels, charcoal, carbon, silica, alumina, Ba₂SO₄, MgO, clay, silicate, naturally occurring zeolite, synthetic zeolite phosphite, aluminate or any combination of these solid support materials.

The heterogeneous catalyst of Formula (A) of the present invention may be formed via process (iii) by a process wherein the transition metal or rare earth metal complex catalyst is anchored onto a solid support through the formation of a strong, transition metal or rare earth metal to polymeric ligand bond. In this process (iii) for the preparation of a heterogeneous catalyst, the ligand L¹, L², L³, L⁴, L⁵, L⁶...L^{p} portion of the transition metal or rare earth metal catalyst of Formula (A) is a polymeric ligand. Such processes for forming heterogeneous catalysts from polymeric ligands are described above under the topic of polymeric ligands and by Y. K. Chung, et. al, in J. of Molecular Catalysis A: Chemical, 174, pp. 151-157, (2001).

In one embodiment of the heterogeneous catalyst process of the present invention, the ratio of the amount of the transition metal or rare earth metal in the catalyst on a metal weight basis to the weight of the solid support material is preferably in the range of from 1x10⁻⁶ part to one part of transition metal or rare earth metal per one part of solid support; more preferably the ratio of the weight of the transition metal or rare earth metal in the catalyst to the weight of the solid support material is in the range of from 1x10⁻⁵ part to 3x10⁻¹ part of transition metal or rare earth metal per one part of solid support; and most preferably, the ratio of the weight of the transition metal or rare earth metal in the catalyst to the weight of the solid support material is in the range from 1x10⁻⁴ part to 1x10⁻¹ part of transition metal or rare earth metal per one part of solid support.

When the catalyst is employed as a supported, heterogeneous catalyst, the allylation reaction can be conducted in a fixed bed or suspended in the liquid reaction mixture; and the reaction can be carried out in a batch or continuous mode.

If desired, when water is employed as a solvent, either as the sole solvent or as a cosolvent, buffering agents such as, for example, alkali or alkaline earth metal bicarbonates such as sodium carbonate or bicarbonate; carbon dioxide; alkali or alkaline earth metal hydrogen phosphates; or alkali or alkaline earth metal borates may be used in the process of the present invention. When water is employed as a solvent, either as the sole solvent or as a cosolvent, with or without a buffering agent, it is preferred that the pH of the reaction mixture be less than 9.

Some additives are useful in promoting allylation of phenolic compounds or mixtures of phenolic compounds with allylating agents. These types of additives may be used in the process of the present invention if desired. For example, some additives may be used in order to replace labile or unstable ligands L, in the metal catalyst represented in Formula (A), including for example, but not limited to, silver tosylate; ammonium tosylate; trimethylsilyl trifluromethane sulfonate; triethylsilyl trifluromethane sulfonate; tri-isopropylsilyl trifluromethane sulfonate; silver trifluromethane sulfonate; ammonium hexaflurophosphonate; silver hexaflurophosphonate.

The amounts of such additives that may be added to the allylation reaction mixture can be varied from 0.1 mole to 10 mole per equivalent of transition metal or rare earth metal.

Suitable solvents which can optionally, but preferably, be employed in the process of the present invention include, for example, water; carbon dioxide; ionic liquids; excess amounts of allyl carboxylate and allyl carbonate; aliphatic, cycloaliphatic and aromatic hydrocarbons; and aliphatic, cycloaliphatic and aromatic halogenated hydrocarbons. Other solvents include, for example, nitro-alkanes and oxygenated hydrocarbons, such as alcohols, ethers, glycols, glycol ethers, esters, and ketones. Any combination of any two or more such solvents can also be used in the present invention. Particularly suitable solvents include, for example, allyl acetate, hexane, octane, cyclohexane, toluene, xylene, chlorobenzene, dichloromethane, 1,2-dichloroethane, acetone, methyl ethyl ketone, ethyl acetate, nitromethane, tetrahydrofuran, ethanol, isopropanol, glymes or any combination thereof.

The preferred solvents are allylacetate, hexane, cyclohexane, toluene, 1,2-dichloroethane, isopropanol, acetone, glycols and glycol ethers or any combination of any two or more such solvents.

The solvents are employed in amounts of from zero to 100 preferably from 0.5 to 20, more preferably from 1 to 10 parts by weight based upon the weight of the phenolic compound.

The aryl allyl ether compound of the present invention is prepared by reacting a phenolic compound with an allylating reagent, such as an allyl carboxylate or an allyl carbonate, in the presence of at least one transition metal or rare earth metal catalyst; and optionally in the presence of at least one buffering agent; and optionally in the presence of at least one salt additive; and further optionally in the presence of one or more suitable solvents.

The allylation reaction is preferably carried out in the presence of a transition metal or rare earth metal catalyst selected from the group consisting essentially of ruthenium, rhenium, molybdenum, tungsten, rhodium, palladium, iridium, platinum, nickel, cobalt, iron, lanthanum, ytterbium, samarium or any combination thereof, with inorganic or organic ligand(s).

Preferably, the present invention allylation reaction is carried out in the presence of at least one ligand or complexing agent, which is strongly or non-replaceably bound directly to the transition metal or rare earth metal of the catalyst.

In the allylation process of the present invention utilizing a heterogeneous catalyst, the amount of heterogeneous catalyst in the reaction mixture may be highly variable dependent on numerous factors, such as, whether the reaction is a batch reaction or a continuous, fixed-bed or fluidized-bed reaction or how much solvent is used. Generally, the weight ratio of heterogeneous catalyst to allylation reaction mixture is 0.1 to 1000 parts of heterogeneous catalyst to 100 parts of allylation reaction mixture. Generally, in batch reactions, the weight ratio of heterogeneous catalyst to allylation reaction mixture is 0.1 to 50 parts of heterogeneous catalyst to 100 parts of allylation reaction mixture. Generally, in continuous, fixed-bed or fluidized-bed reactions, the weight ratio of heterogeneous catalyst to allylation reaction mixture is 100 to 1000 parts of heterogeneous catalyst to 100 parts of allylation mixture.

In the allylation process of the present invention utilizing a homogeneous catalyst, a preferred molar ratio of the transition metal or rare earth metal catalyst to phenolic compound present in the reaction mixture is from 1x10⁻⁶ equivalents to 0.1 equivalents of catalyst per one equivalent of phenolic compound; a more preferred molar ratio of the transition metal or rare earth metal catalyst to phenolic compound present in the reaction mixture is from 1x10⁻⁶ equivalents to 0.01 equivalents of catalyst per one equivalent of phenolic compound; the most preferred molar ratio of the transition metal or rare earth metal catalyst to phenolic compound present in the reaction mixture is from 1x10⁻⁶ equivalents to 0.001 equivalents of catalyst per one equivalent of phenolic compound.

The allylation reaction may be conducted at a temperature of from 10°C to 200°C, preferably from 50°C to 150°C, more preferably from 70°C to 110°C and for a time sufficient to essentially complete the reaction, usually for 0.1 to 72 hours, preferably from 0.1 to 48 hours, and more preferably from 0.1 to 24 hours. Optimum allylation reaction temperatures and times for particular reactants will vary depending upon the reactivity of the particular reactants, solvents and catalysts employed. Particular pressures are not deemed to be determinative of any appreciative affects on the process; however, pressures should be employed which preferably will keep the reaction in the liquid phase. For example, a pressure of from 50 mmHg to 2 atmospheres may be used in the process of the present invention.

Once the allylation has been carried out to the desired degree of conversion, the aryl allyl ether product may be separated and recovered from the reaction mixture using any appropriate techniques or suitable means. A particularly suitable means may include, for example, filtration or decantation from the catalyst if the transition metal or rare earth metal catalyst and its associated complexing agent are present as a heterogeneous phase in the reaction mixture. In instances where the transition metal or rare earth metal catalyst and its associated complexing agent are present as a homogeneous phase in the reaction mixture, the catalyst may be removed, for instance, by membrane filtration techniques such as described by D. Turlan, et. al, in Chemical Communications, pp. 2608-2609, (2001); by nanofiltration techniques, such as described by A. G. Livingston et. al, in Tetrahedron Letters, 42, pp. 8219-8222, (2001); or by extraction techniques such as described in U.S. Patent Nos. 5,874,639; 5,874,640; 5,892,119; and 5,917,095.

Once the catalyst is separated from the allylation reaction mixture, the allylation reaction product may be separated and recovered from the reaction mixture using any appropriate techniques such as filtration, washing, fractional distillation, extractive distillation, liquid-liquid extraction, solid-liquid extraction and crystallization, or any combination of these methods. For example, one product separation scheme could involve removal of all volatiles, such as solvent and unreacted allylating agent, from the reaction (mixture by distillation or evaporation. Then recovering the carboxylic acid by product by distillation or extraction to recover the desired allyl derivative product as a bottom product. The carboxylic acid by-product of allylation with an allyl carboxylate recovered using these techniques may be recycled to allyl carboxylate manufacture.

If it is required to obtain the allyl derivative compound in a more purified state, then the allyl derivative compound may be further purified by suitable means such as distillation, crystalization, sublimation, or extraction.

These example schemes for separation of an allylation reaction mixture are intended only to describe some of many possible separation schemes. The separation scheme of choice will depend on many factors such as the boiling points, melting points, thermal stabilities, and solubilities of the various components of the allylation reaction mixture.

The heterogeneous and homogeneous catalysts of the present invention may be reusable after each allylation reaction. The catalyst may be separated and reused without additional treatment or the catalyst may be regenerated by any of several methods, such as through washing with certain non-reactive solvents or dilute acid or base, well-known to those skilled in the art.

It is also preferred that with certain combinations of carboxylic acid and phenolic compounds that the aryl allyl ether compound is formed in situ during the allyl carboxylate synthesis step.

The aryl allyl ether compounds made by the process of the present invention are useful products directly in their allyl form, for instance, the compounds may be used as monomers in polymerization reactions, such as taught in European Patent EP 458240 with bismaleimide compositions and in European Patent EP 269392 A2 with cyanate ester compositions.

Additionally, the allyl derivatives made by the process of the present invention may optionally be directly converted, for example by epoxidation, to useful commercial products, such as epoxy resins, as described in U.S. Patent Nos. 5,578,740 and 6,087,513.

The aryl allyl ethers made by the process of the present invention may optionally be further reacted, for example by dihydroxylation, to make new derivatives, for example α-dihydroxy derivatives, useful in preparing intermediates, such as carbonate intermediates or α-halohydrin intermediates, which can in turn be converted to useful commercial products, such as epoxy resins, such as disclosed in U.S. Patent Application Serial No. 09/899,409, Attorney Docket No. C-60991; and U.S. Patent Nos. 6,172,182 B1 and 6,001,954. The intermediate may also be converted to useful commercial products, such as fine chemicals, as described in U.S. Patent No. 4,871,855 and in Japanese Patent JP 05,271,270.

The following examples are illustrative of the present invention, but are not to be construed as to affecting the scope thereof in any manner. The following comparative examples employ the method described in U.S. Patent No. 5,578,740.

The following general procedure and reaction equipment were used in the examples and comparative examples listed below:

A 100-ml, round bottom, four-necked, glass reactor equipped with a cooling condenser, a thermometer, a magnetic stirrer, a nitrogen inlet and a heating lamp with thermo-controller was employed. At the ambient temperature (25°C) under a nitrogen atmosphere, to the reactor were added catalyst, phenols, allyl acetate (commercially available from Aldrich Chemical Co.), solvent or other ingredient as indicated in the examples. The resultant reaction mixture was stirred for a period of time at the temperature as indicated, and the mixture of reaction product was then subjected to gas chromatography analysis or to gas chromatography/mass spectrometry analysis for the conversion of phenol or the yield of allyl ether of phenol.

### Example 1

In this example, phenyl allyl ether was prepared from phenol and allyl acetate using a homogeneous ruthenium catalyst and allyl acetate as the solvent.

A mixture of 4.7 g (0.05 mole) of phenol, 0.363 g (1.0 percent equiv. mole) of chloro(cyclopentadienyl)bis(triphenylphosphinyl) ruthenium(II), and 25 g of allyl acetate (0.25 mole) under a nitrogen atmosphere is stirred at 95°C for 4 hours. After 4 hours, gas chromatography analysis shows that the conversion of phenol to phenyl allyl ether is 74 percent.

### Comparative Example A

In this example, phenyl allyl ether was prepared from phenol and allyl alcohol. Allyl alcohol is not an allylating agent of the present invention.

A mixture of 4.7 g (0.05 mole) of phenol, 0.182 g (0.005 equivalents) of chloro(cyclopentadienyl)bis(triphenylphosphinyl) ruthenium(II), and 14.52 g of allyl alcohol (0.25 mole) under a nitrogen atmosphere is stirred at 95°C. After 4 hours, gas chromatography analysis shows that the conversion of phenol to phenyl allyl ether is only 11 percent, and the majority of the reacted allyl alcohol has converted into a self condensation product methylpentanal confirmed by gas chromatography/mass spectrometry analysis.

### Comparative Example B

In this example, preparation of phenyl allyl ether from phenol and allyl acetate was attempted using a homogeneous ruthenium catalyst outside the scope of the present invention.

A mixture of 4.7 g (0.05 mole) of phenol, 0.376 g (1.0 percent equiv. mole) of dicarbonyldichloro-bis(triphenylphosphinyl) ruthenium(II), Ru(CO)₂(PPh₃)₂(Cl)₂, and 25 g of allyl acetate (0.25 mole) under a nitrogen atmosphere is stirred at 105°C. After 4 hours, gas chromatography analysis shows that the conversion to phenyl allyl ether is <1 percent.

### Example 2

In this example, phenyl allyl ether was prepared from phenol and allyl acetate using a homogeneous ruthenium catalyst in the presence of a solvent.

A mixture of 4.7 g (0.05 mole) of phenol, 0.182 g (0.5 percent equiv. mole) of chloro(cyclopentadienyl)bis(triphenylphosphinyl) ruthenium(II), and 10 g of allyl acetate (0.10 mole) in 15 g tetrachloroethane under a nitrogen atmosphere is stirred at 95°C. After 4 hours, gas chromatography analysis shows that the conversion to phenyl allyl ether is 45 percent.

### Example 3

In this example, phenyl allyl ether was prepared from phenol and allyl acetate using a homogeneous ruthenium catalyst in the presence of a solvent.

A mixture of 4.7 g (0.05 mole) of phenol, 0.363 g (1 percent mole) of chloro(cyclopentadienyl)bis(triphenylphosphinyl) ruthenium(II), and 10 g of allyl acetate (0.1 mole) in 15 g tetrachloroethane under a nitrogen atmosphere is stirred at 95°C. After 4 hours, gas chromatography analysis shows that the conversion to phenyl allyl ether is 61 percent.

### Example 4

In this example, phenyl allyl ether was prepared from phenol and allyl acetate using a homogeneous ruthenium catalyst in the presence of a solvent.

A solution of 4.7 g (0.05 mole) of phenol, 0.363 g (1 percent mole) of chloro(cyclopentadienyl)bis(triphenylphosphinyl) ruthenium(II), and 10 g of allyl acetate (0.1 mole) in 15 g tert-amyl alcohol under a nitrogen atmosphere is stirred at 95°C. After 4 hours, gas chromatography analysis shows that the conversion to phenyl allyl ether is 55 percent.

### Example 5

In this example, phenyl allyl ether was prepared from phenol and allyl acetate using a homogeneous ruthenium catalyst in the presence of a solvent.

A solution of 2.82 g (0.03 mole) of phenol, 0.09 g (0.5 percent mole) of chloro(cyclopentadienyl)[1,2-bis(diphenylphosphinyl)ethane]ruthenium(II), and 6 g of allyl acetate (0.06 mole) in 10 g tetrachloroethane under a nitrogen atmosphere is stirred at 95°C. After 4 hours, gas chromatography analysis shows that the conversion to phenyl allyl ether is 21 percent.

### Example 6

In this example, 2,6-dimethylphenyl allyl ether was prepared from 2,6-dimethylphenol and allyl acetate using a homogeneous ruthenium catalyst and allyl acetate as the solvent.

A mixture of 3.05 g (0.025 mole) of 2,6-dimethylphenol, 0.182 g (1 percent mole) of chloro(cyclopentadienyl)bis(triphenylphosphinyl) ruthenium(II), and 12.5 g of allyl acetate (0.125 mole) under a nitrogen atmosphere is stirred at 95°C. After 4 hours, gas chromatography analysis shows that the conversion to 2,6-dimethylphenyl allyl ether is 30 percent.

### Example 7

In this example, bisphenol A diallyl ether was prepared from bisphenol A and allyl acetate using a homogeneous ruthenium catalyst with allyl acetate as solvent.

A mixture of 2.85 g (0.0125 mole) of bisphenol A, 0.363 g (2 percent mole) of chloro(cyclopentadienyl)bis(triphenylphosphinyl) ruthenium(II), and 25 g of allyl acetate (0.25 mole) under a nitrogen atmosphere is stirred at 95°C. After 6 hours, gas chromatography analysis show that the conversion of bisphenol A is 96 percent; the phenolic OH conversion is 89 percent; and the ratio of diallyl ether to monoallyl ether to bisphenol A is 82:14:4.

### Example 8

In this example, the diallyl ether of bisphenol A was prepared from bisphenol A and allyl acetate using a homogeneous ruthenium catalyst with allyl acetate as solvent.

A solution of 2.85 g (0.0125 mole) of bisphenol A, 0.182 g (1 percent mole) of chloro(cyclopentadienyl)[1,2-bis(diphenylphosphinyl)ethane]ruthenium(II), and 12.5 g of allyl acetate (0.125 mole) under a nitrogen atmosphere is stirred at 95°C. After 4 hours, gas chromatography analysis shows that the conversion to bisphenol A diallyl ether is 56 percent.

### Example 9

In this example, the diallyl ether of bisphenol A was prepared from bisphenol A and allyl acetate using a homogeneous ruthenium catalyst with water as solvent.

A solution of 2.85 g (0.0125 mole) of bisphenol A, 0.182 g (1 percent mole) of chloro(cyclopentadienyl)[1,2-bis(diphenylphosphinyl)ethane]ruthenium(II), and 12.5 g of allyl acetate (0.125 mole) in 6.3 g water under a nitrogen atmosphere is stirred at 95°C. After 4 hours, gas chromatography analysis shows that the conversion to bisphenol A diallyl ether is 25 percent.

### Example 10

In this example, phenyl allyl ether was prepared from phenol and allyl acetate using a homogeneous iridium catalyst.

A solution of 4.70 g (0.05 mole) of phenol, 0.248 g (1.0 percent mole) of [Ir(COD)₂]⁺ BF₄⁻ and 10 g of allyl acetate (0.10 mole) in 50 g tetrachloroethane under a nitrogen atmosphere is stirred at 95°C. After 4 hours, gas chromatography analysis shows that the conversion to phenyl allyl ether is 29 percent.

### Example 11

In this example, phenyl allyl ether was prepared from phenol and allyl acetate using a palladium catalyst and ten equivalents of a phosphine moiety in the form of a phosphorous heteroatom-containing polymeric ligand.

A solution of 2.35 g (0.025 mole) of phenol, 0.06 g (1.0 percent mole) of palladium acetate, Pd(OAc)₂, 0.80 g crosslinked styrene polymer supported triphenylphosphine (10 equivalents per Pd equivalent) and 15 g of allyl acetate (0.15 mole under a nitrogen atmosphere is stirred at 95°C. After 4 hours, gas chromatography analysis shows that the conversion to phenyl allyl ether is 82 percent.

### Comparative Example C

In this example, phenyl allyl ether was prepared from phenol and allyl acetate using a palladium catalyst and ten equivalents (based on Pd) of a monodentate phosphine ligand.

A solution of 2.35 g (0.025 mole) of phenol, 0.06 g (1.0 percent mole) of palladium acetate, Pd(OAc)₂, 0.72 g triphenylphosphine (10 equivalents per Pd equivalent) and 15 g of allyl acetate (0.15 mole) under a nitrogen atmosphere is stirred at 95°C. After 4 hours, gas chromatography analysis shows that the conversion to phenyl allyl ether is 52 percent.

### Example 12

In this example, phenyl allyl ether was prepared from phenol and allyl acetate using a palladium catalyst and six equivalents (based on Pd) of a phosphine moiety in the form of a phosphorous heteroatom-containing polymeric ligand.

A solution of 2.35 g (0.025 mole) of phenol, 0.06 g (1.0 percent mole) of palladium acetate, Pd(OAc)₂, 0.48 g crosslinked styrene polymer supported triphenylphosphine (6 equivalents per Pd equivalent) and 15 g of allyl acetate (0.15 mole) under a nitrogen atmosphere is stirred at 95°C. After 4 hours, gas chromatography analysis shows that the conversion to phenyl allyl ether is 79 percent.

### Comparative Example D

In this example, phenyl allyl ether was prepared from phenol and allyl acetate using a palladium catalyst and six equivalents (based on Pd) of a monodentate phosphine ligand.

A solution of 2.35 g (0.025 mole) of phenol, 0.06 g (1.0 percent mole) of palladium acetate, Pd(OAc)₂, 0.435 g triphenylphosphine (6 equivalents per Pd equivalents) and 15 g of allyl acetate (0.15 mole) under a nitrogen atmosphere is stirred at 95°C. After 4 hours, gas chromatography analysis shows that the conversion to phenyl allyl ether is 47 percent.

### Comparative Example E

In this example, phenyl allyl ether was prepared from phenol and allyl acetate using a palladium catalyst and six equivalents (based on Pd) of phosphorous as a didentate phosphine ligand.

A solution of 2.35 g (0.025 mole) of phenol, 0.06 g (1.0 percent mole) of palladium acetate, Pd(OAc)₂, 0.30 g Ph₂PCH₂CH₂PPh₂ (6 equivalents per Pd equivalent) and 15 g of allyl acetate (0.15 mole) under a nitrogen atmosphere is stirred at 95°C. After 4 hours, gas chromatography analysis shows that the conversion to phenyl allyl ether is 10 percent.

### Comparative Example F

In this example, phenyl allyl ether was prepared from phenol and allyl acetate using a palladium catalyst and six equivalents (based on Pd) of phosphorous as a didentate phosphine ligand.

A solution of 2.35 g (0.025 mole) of phenol, 0.06 g (1.0 percent mole) of palladium acetate, Pd(OAc)₂, 0.47 g 2,2'-bis(diphenylphosphinyl)-1,1'-binaphthyl (6 equivalents per Pd equivalent) and 15 g of allyl acetate (0.15 mole) under a nitrogen atmosphere is stirred at 105°C. After 4 hours, gas chromatography analysis shows that the conversion to phenyl allyl ether is 12 percent.

### Example 13

In this example, phenyl allyl ether was prepared from phenol and allyl acetate using a palladium catalyst and two equivalents of a phosphine moiety in the form of a phosphorous heteroatom-containing polymeric ligand. A solution of 2.35 g (0.025 mole) of phenol, 0.06 g (1.0 percent mole) of palladium acetate, Pd(OAc)₂, 0.16 g crosslinked styrene polymer supported triphenylphosphine (2 equivalents per Pd equivalent) and 15 g of allyl acetate (0.15 mole) under a nitrogen atmosphere is stirred at 95°C. After 4 hours, gas chromatography analysis shows that the conversion to phenyl allyl ether is 65 percent.

### Comparative Example G

In this example, phenyl allyl ether was prepared from phenol and allyl acetate using a palladium catalyst and four equivalents (based on Pd) of phosphorous as a didentate phosphine ligand.

A solution of 2.35 g (0.025 mole) of phenol, 0.06 g (1.0 percent mole) of palladium acetate, Pd(OAc)₂, 0.21 g Ph₂PCH₂CH₂CH₂PPh₂ (4 equivalents per Pd equivalent) and 15 g of allyl acetate (0.15 mole) under a nitrogen atmosphere is stirred at 95°C. After 4 hours, gas chromatography analysis shows that the conversion to phenyl allyl ether is 17 percent. After an additional 2 hrs at 105°C, phenyl allyl ether yield is 23 percent.

### Example 14

In this example, bisphenol A diallyl ether was prepared from bisphenol A and allyl acetate using a palladium catalyst and six equivalents of a phosphine moiety in the form of a phosphorous heteroatom-containing polymeric ligand.

A solution of 2.85 g (0.0125 mole) of bisphenol A, 0.06 g (1.0 percent mole) of palladium acetate, Pd(OAc)₂, 0.48 g crosslinked styrene polymer supported triphenylphosphine (6 equivalents per Pd equivalent) and 15 g of allyl acetate (0.15 mole) under a nitrogen atmosphere is stirred at 95°C. After 4 hours, gas chromatography analysis shows that the conversion of bisphenol A is 91 percent, and the ratio of bisphenol A to mono allyl ether of bisphenol A to diallyl ether of bisphenol A is 9:60:31.

### Example 15

In this example, bisphenol A diallyl ether was prepared from bisphenol A and allyl acetate using a palladium catalyst and ten equivalents of a phosphine moiety in the form of a phosphorous heteroatom-containing polymeric ligand.

A solution of 2.85 g (0.0125 mole) of bisphenol A, 0.12 g (2.0 percent mole) of palladium acetate, Pd(OAc)₂, 0.80 g crosslinked styrene polymer supported triphenylphosphine (10 equivalents per Pd equivalent) and 15 g of allyl acetate (0.15 mole) under a nitrogen atmosphere is stirred at 95°C. After 8 hours, gas chromatography analysis shows that the conversion of bisphenol A is 92 percent, and the ratio of bisphenol A to mono allyl ether of bisphenol A to diallyl ether of bisphenol A is 8:46:46.

## Claims

1. A process for preparing an aryl allyl ether comprising reacting (a) a phenolic compound with (b) an allyl acetate in the presence of (c) an allylation catalyst selected from a ruthenium or rare earth metal catalyst complex comprising at least one stable ligand containing a substituted aromatic-containing moiety, wherein the aromatic-containing ligand is cyclopentadienyl (Cp), pentamethylcyclopentadienyl (Cp*), indenyl (In), or 9-fluorenyl (FI), or a substituted olefinic-containing ligand, wherein the olefinic moiety is a cyclooctadienyl (COD) or butyldienyl moiety or substituted olefinic-containing ligands.

2. The process of Claim 1 wherein the ruthenium or rare earth metal catalyst complex with ligand is represented by partial structural Formula E, F, or G: 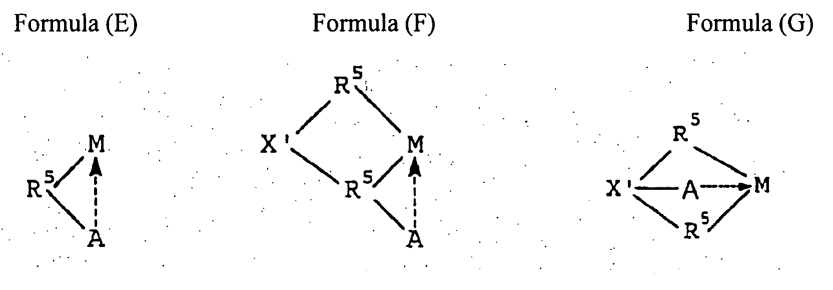 wherein M is ruthenium or rare earth metal; R⁵ is a monodentate olefinic or an aromatic-containing ligand, and X' is a moiety bridging or linking the R⁵ groups together such that the R⁵-X'- R⁵ structure becomes a bidentate ligand, wherein each R⁵ may be the same or a different group; and A is an ancillary group attached to the R⁵ or X' group, wherein A may be an aliphatic, cycloaliphatic, aromatic, or combination thereof moiety having one or more heteroatoms capable of forming one or more coordination bonds with M.

3. The process of Claim 1 or 2 wherein the phenolic compound is represented by the following formula:
(R¹)ₓ Ar(OR²)_{y}
wherein x is from 0 to 750; y is from 1 to 150; Ar is an aromatic moiety; R¹ is a group substituted for a hydrogen atom on the aromatic ring(s) of the Ar moiety; and R² is a hydrogen atom.

4. The process of any one of the preceding claims wherein the phenolic compound is bisphenol A.

5. The process of any one of Claims 1 to 3 wherein the aryl allyl ether compound is represented by the following formula
(R¹)ₓ Ar(OR²)_{y}
wherein x is from 0 to 750; y is from 1 to 150; Ar is an aromatic moiety; R¹ is a group substituted for a hydrogen atom on the aromatic ring(s) of the Ar moiety; and R² is a propenyl-containing moiety.

6. The process of Claim 5 wherein the propenyl-containing moiety is -CH₂CH=CH₂.

7. The process of any one of Claims 1 to 4 wherein the aryl allyl ether compound is a diallyl ether of bisphenol A.

8. The process of any one of the preceding claims including a solvent.

9. The process of any one of the preceding claims wherein the pH of the reaction mixture is less than 9.

10. The process of any one of the preceding claims wherein the reaction is conducted at a temperature of from 10°C to 200°C.

11. The process of any one of the preceding claims wherein the ruthenium or rare earth metal catalyst is a homogeneous catalyst, or a heterogeneous catalyst which includes a solid support material.

12. The process of any one of the preceding claims wherein the equivalent ratio of allylating reagent to phenolic compound is 0.1 to 500 equivalents of allylating reagent to 1 equivalent of phenolic hydroxy.

13. A process for the preparation of an epoxy-containing compound comprising epoxidizing the aryl allyl ether compound made according to any one of the preceding claims.
